# FASCICULE DE BREVET EUROPEEN

(11) **EP 1 125 942 B1**
(45) Date de publication et mention de la délivrance du brevet: **09.04.2003**
(21) Numéro de dépôt: 01400409.7
(22) Date de dépôt: 16.02.2001
(51) Int. Cl.: C07F 9/60, A61K 31/675, A61P 25/00

(54) **Dérivés d'acides 6-amino ou 6-hydrazinosulfonyl-3-quinolinyl phosphoniques, leur procédé de préparation et les compositions pharmaceutiques qui les contiennent**
6-Amino oder 6-Hydrazinosulfonyl-3-quinolylphosphonsäurederivate, Verfahren zu ihrer Herstellung und sie enthaltende Arzneimittel
Derivatives of 6-amino or 6-hydrazinosulfonyl-3-quinolyl phosphonic acids, process for their preparation and pharmaceutical compositions containing them

(30) Priorité: 18.02.2000 FR 0002012
(43) Date de publication de la demande: 22.08.2001
(73) Titulaire: LES LABORATOIRES SERVIER, 92200 Neuilly sur Seine (FR)
(72) Inventeur: Cordi, Alex, 92150 Suresnes (FR); Desos, Patrice, 92400 Courbevoie (FR); Lestage, Pierre, 78170 La Celle Saint Cloud (FR)

(56) Documents cités:
- EP-A- 0 542 609
- EP-A- 0 640 612
- EP-A- 0 818 449
- US-A- 5 342 946

## Description

La présente invention concerne de nouveaux dérivés d'acides 6-amino ou 6-hydrazinosulfonyl-3-quinolinyl phosphoniques, leur procédé de préparation et les compositions qui les contiennent.

L'art antérieur décrit des composés capables de s'opposer aux effets excitateurs et toxiques des aminoacides excitateurs (AAE) en bloquant l'activation initiale du récepteur à l'AMPA (acide α-amino-3-hydroxy-5-méthyl-4-isoxazole-propionique) /kainate (EP0640612). Leur utilité est ainsi reconnue pour inhiber les phénomènes pathologiques, notamment neurotoxiques liés à l'hyperactivation des voies de neurotransmission aux aminoacides excitateurs. Cependant, ces composés présentent des problèmes graves de néphrotoxicité comme cela a pu être montré par ailleurs pour d'autres antagonistes non-NMDA (N-méthyl-D-aspartate) de référence comme le 6-nitro-7-sulfamoyl-benzo[*f*]quinoxaline-2,3-dione (NBQX) par exemple (Journal of Cerebral Blood Flow and Metabolism, 1994, 14, 251-261).

La demanderesse a découvert de nouveaux composés qui présentent des propriétés d'antagonistes non-NMDA plus puissants que ceux de l'art antérieur avec une néphrotoxicité associée bien moindre. Ces composés sont donc nouveaux et sont de puissants agents thérapeutiques potentiels pour le traitement aigu mais également chronique des maladies neurologiques et psychiques impliquant ces aminoacides comme les maladies dégénératives telles que l'accident vasculaire cérébral, le traumatisme cérébral ou spinal, l'épilepsie, les maladies neurodégénératives chroniques telles que la maladie d'Alzheimer, la schizophrénie, la sclérose amyotrophique latérale ou la chorée de Huntington.

Plus particulièrement, la présente invention concerne les composés de formule (I) : 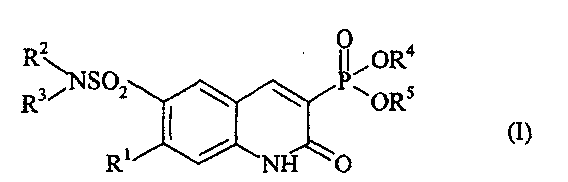 dans laquelle :
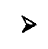 R¹ représente un atome d'halogène ou un groupement trifluorométhyle,
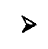 R² représente un atome d'hydrogène ou un groupement alkyle ou cycloalkyle,
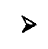 R³ représente un groupement alkyle, cycloalkyle, aryle, arylalkyle, alkoxy, aryloxy, arylalkoxy, hydroxy, aminoalkyle, alkylaminoalkyle, dialkylaminoalkyle, alkylcarbonyle, arylcarbonyle, arylalkylcarbonyle, ou NHR⁶ (où R⁶ représente un atome d'hydrogène ou un groupement alkyle, cycloalkyle, aryle, alkylcarbonyle, arylcarbonyle, arylalkyle ou arylalkylcarbonyle),
ou R² et R³ forment ensemble, avec l'atome d'azote qui les porte un cycle à 5 ou 6 atomes de carbone dans lequel un des atomes de carbone peut être remplacé par un atome d'oxygène, d'azote ou de soufre ou par un groupement SO, SO₂, ou NRₐ (dans lequel Rₐ représente un atome d'hydrogène ou un groupement alkyle, cycloalkyle, aryle ou arylalkyle),
> R⁴ et R⁵, identiques ou différents, représentent un atome d'hydrogène ou un groupement alkyle, cycloalkyle, aryle, arylalkyle ou un groupement 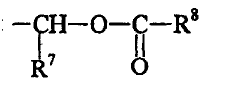 (dans lequel R⁷ et R⁸, identiques ou différents, représentent un atome d'hydrogène ou un groupement alkyle, cycloalkyle ou aryle),
étant entendu que :
- par alkyle on entend un groupement alkyle contenant 1 à 6 atomes de carbone, linéaire ou ramifié,
- par alkoxy on entend un groupement alkoxy contenant 1 à 6 atomes de carbone, linéaire ou ramifié,
- par cycloalkyle on entend un groupement alkyle cyclique contenant de 3 à 8 atomes de carbone,
- par aryle on entend les groupements phényle ou naphtyle, ces groupements pouvant être non substitués ou substitués par 1 à 3 groupements choisis parmi alkyle, cycloalkyle, hydroxy, alkoxy, amino, alkylamino, dialkylamino, cyano, nitro, polyhalogénoalkyle, SO₂NR⁹R¹⁰ (où R⁹ et R¹⁰, identiques ou différents, représentent un atome d'hydrogène ou un groupement alkyle, cycloalkyle ou aryle), ou atomes d'halogène,
leurs énantiomères et diastéréoisomères ainsi que leurs sels d'addition à un acide ou à une base pharmaceutiquement acceptable.

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, méthanesulfonique, camphorique, oxalique, etc...

Parmi les bases pharmaceutiquement acceptables, on peut citer à titre non limitatif l'hydroxyde de sodium, l'hydroxyde de potassium, la triéthylamine, la tertbutylamine, etc...

Les composés préférés de l'invention sont les composés de formule (I) pour lesquels R¹ représente un atome de chlore.

Les groupements R³ préférés sont le groupement alkyle (comme par exemple méthyle ou propyle), le groupement dialkylaminoalkyle (comme par exemple diméthylaminoéthyle), le groupement aryle (comme par exemple phényle), arylcarbonyle (comme par exemple benzoyle) ou le groupement NHCOR dans lequel R représente un groupement alkyle ou aryle.

Un aspect avantageux de l'invention concerne les composés de formule (I) pour lesquels R² représente un atome d'hydrogène ou un groupement alkyle.

Lorsque R² représente un atome d'hydrogène, R³ représente avantageusement un groupement cycloalkyle, aryle, arylalkyle, alkoxy, aryloxy, arylalkoxy, hydroxy, aminoalkyle, alkylaminoalkyle, dialkylaminoalkyle, alkylcarbonyle, arylcarbonyle, arylalkylcarbonyle, ou NHR⁶.

Lorsque R² représente un groupement alkyle, R³ représente avantageusement un groupement alkyle.

L'atome d'hydrogène est la valeur préférée pour les substituants R⁴ et R⁵.

Encore plus particulièrement, l'invention concerne les composés de formule (I) qui sont :
* l'acide 7-chloro-2-oxo-6-[(*n*-propylamino)sulfonyl]-1,2-dihydro-3-quinolinyl phosphonique,
* l'acide 7-chloro-6-[(méthylamino)sulfonyl]-2-oxo-1,2-dihydro-3-quinolinyl phosphonique,
* l'acide 6-(anilinosulfonyl)-7-chloro-2-oxo-1,2-dihydro-3-quinolinyl phosphonique,
* l'acide 6-[(2-benzoylhydrazino)sulfonyl]-7-chloro-2-oxo-1,2-dihydro-3-quinolinyl phosphonique,
* l'acide 7-chloro-6-[(2-{4-[(di-n-propylamino)sulfonyl]benzoyl}hydrazino)sulfonyl]-2-oxo-1,2-dihydro-3-quinolinyl phosphonique,
* le bromhydrate de l'acide 7-chloro-6-({[2-(diméthylamino)éthyl)amino}sulfonyl)-2-oxo-1,2-dihydro-3-quinolinyl phosphonique.

Les énantiomères, diastéréoisomères ainsi que les sels d'addition à un acide ou à une base pharmaceutiquement acceptables des composés préférés de l'invention font partie intégrante de l'invention.

L'invention s'étend également au procédé de préparation des composés de formule (I) caractérisé en ce que l'on utilise comme produit de départ le composé de formule (II) : 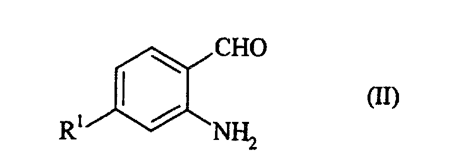 dans laquelle R¹ est tel que défini dans la formule (I),
sur lequel on condense en présence de base comme la pyridine par exemple un composé de formule (III) : 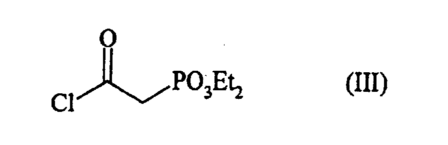 pour conduire au composé de formule (IV) : 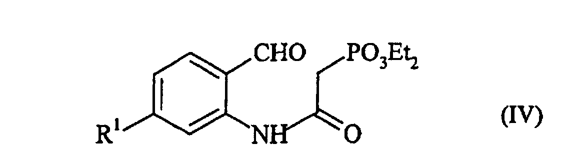 dans laquelle R¹ est tel que défini précédemment,
que l'on cyclise en présence d'une quantité catalytique de pipéridine pour obtenir le composé de formule (V) 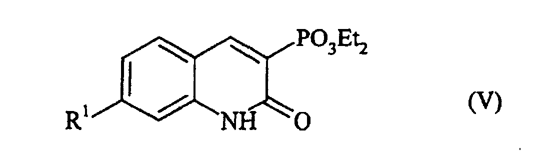 dans laquelle R¹ est défini comme précédemment,
que l'on soumet à un mélange d'acide nitrique et d'acide sulfurique pour conduire au composé de formule (VI) : 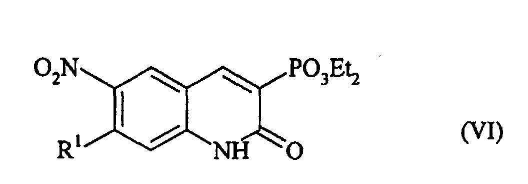 dans laquelle R¹ est tel que défini précédemment,
qui est réduit en utilisant du charbon palladié en présence d'hydrogène ou du Fer en milieu hydroalcoolique pour conduire au composé de formule (VII) : 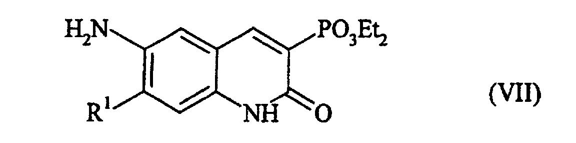 dans laquelle R¹ est défini comme précédemment,
qui est soumis, après transformation en sel de diazonium correspondant, à l'action de dioxide de soufre en présence de CuCl₂ pour conduire au composé de formule (VIII) : 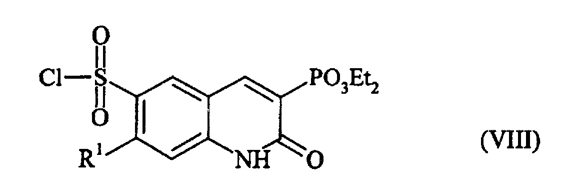 dans laquelle R¹ est tel que défini précédemment,
sur lequel on condense un composé de formule HNR²R³ dans lequel R² et R³ sont tels que définis dans la formule (I) pour conduire au composé de formule (I/a), cas particulier des composés de formule (I) : 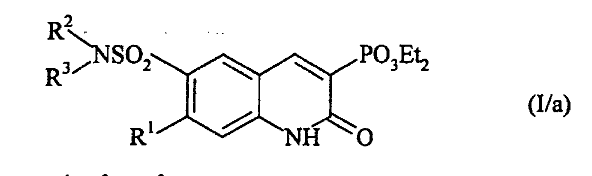 dans laquelle R¹, R² et R³ sont tels que définis précédemment,
qui est partiellement ou totalement déprotégé en présence de bromure de triméthylsilane par exemple pour conduire au composé de formule (I/b), cas particulier des composés de formule (I) : 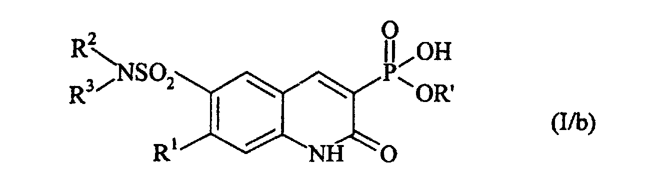 dans laquelle R¹, R² et R³ sont tels que définis précédemment et R' représente un atome d'hydrogène ou un groupement éthyle,
sur lequel on peut condenser un composé de formule (IX) :

R"-Cl (IX)

dans laquelle R" représente un groupement alkyle, aryle, arylalkyle ou 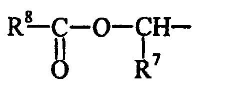 (où R⁷ et R⁸ sont définis comme dans la formule (I)), pour obtenir le composé de formule (I/c), cas particulier des composés de formule (I) : 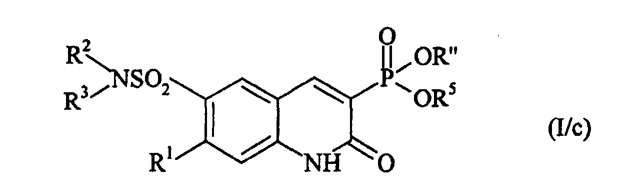 dans laquelle R¹, R², R³, R⁵ et R" sont tels que définis précédemment,
les composés de formule (I/a) à (I/c) formant l'ensemble des composés de formule (I), et pouvant être purifiés selon une technique classique de séparation, que l'on transforme, si on le souhaite en leurs sels d'addition à un acide ou à une base pharmaceutiquement acceptable et dont on sépare éventuellement les isomères selon une technique classique de séparation.

Les composés de l'invention présentent des propriétés pharmacologiques tout à fait intéressantes puisqu'ils sont de puissants inhibiteurs du récepteur AMPA, de surcroît sélectifs puisqu'ils ne touchent pas le récepteur NMDA et sont donc dépourvus de tous les effets secondaires décrits pour les antagonistes NMDA, et surtout dépourvus de la néphrotoxicité associée à nombre d'antagonistes AMPA/non-NMDA. L'utilisation de ces composés en tant qu'inhibiteurs des phénomènes pathologiques liés à l'hyperactivation des voies de neurotransmissions aux aminoacides excitateurs sera ainsi particulièrement appréciée dans les traitements aigus et surtout chroniques des maladies neurologiques et psychiques impliquant ces aminoacides comme les maladies dégénératives telles que l'accident vasculaire cérébral, le traumatisme cérébral ou spinal, l'épilepsie, les maladies neurodégénératives chroniques telles que la maladie d'Alzheimer, la schizophrénie, la sclérose amyotrophique latérale ou la chorée de Huntington.

La présente invention a également pour objet les compositions pharmaceutiques renfermant comme principe actif au moins un composé de formule (I) seul ou en combinaison avec un ou plusieurs excipients pharmaceutiquement acceptables.

Parmi les compositions pharmaceutiques selon l'invention, on pourra citer plus particulièrement celles qui conviennent pour l'administration orale, parentérale, nasale, per ou transcutanée, rectale, perlinguale, oculaire ou respiratoire et notamment les comprimés simples ou dragéifiés, les comprimés sublinguaux, les sachets, les paquets, les gélules, les glossettes, les tablettes, les suppositoires, les crèmes, les pommades, les gels dermiques et les ampoules buvables ou injectables.

La posologie varie selon le sexe, l'âge et le poids du patient, la voie d'administration, la nature de l'indication thérapeutique, ou des traitements éventuellement associés et s'échelonne entre 50 mg et 1 g par 24 heures en 1 ou plusieurs prises.

Les exemples suivants illustrent l'invention et ne la limitent en aucune façon.

### EXEMPLE 1 : Acide 7-chloro-2-oxo-6-[(n-propylamino)sulfonyl]-1,2-dihydro-3-quinolinyl phosphonique diéthyl ester

### Stade A : Acide [(5-chloro-2-formyl-phénylcarbamoyl)méthyl]phosphonique diéthyl ester

A une solution de 2-amino-4-chloro-benzaldéhyde (6,18 g, 39,7 mmol) dans 170 ml de toluène anhydre on rajoute la pyridine (3,7 ml, 45,7 mmol) puis goutte à goutte une solution d'acide chlorocarbonylméthyl-phosphonique diéthyl ester (9,8 g, 45,7 mmol) dans 15 ml de toluène anhydre en maintenant la réaction à une température inférieure à 30°C. A la fin de l'addition, on agite 1 heure à température ambiante. La réaction est lavée plusieurs fois à l'eau puis avec une solution HCI 1N, puis de nouveau à l'eau. On termine par un lavage avec une solution aqueuse de NaCl saturée. La phase organique est séchée sur MgSO₄, on filtre, évapore et on obtient le produit brut attendu sous forme d'une huile orange. Le produit brut est engagé dans l'étape suivante.

### Stade B : Acide (7-chloro-2-oxo-1,2-dihydro-3-quinolinyl)phosphonique diéthyl ester

Dans un ballon surmonté d'un Dean-Stark on agite au reflux sous forte agitation pendant 4 heures la totalité du composé obtenu au stade A en solution dans 300 ml de toluène et 0,3 ml de pipéridine. On laisse cristalliser à température ambiante et on filtre le solide jaune pale obtenu.
*Point de fusion : 210-213°C*

| *Microanalyse élémentaire :* | | | | |
|---|---|---|---|---|
| | *C%* | *H%* | *N%* | *Cl%* |
| *calculé* | *49,46* | *4,79* | *4,44* | *11,23* |
| *trouvé* | *49,77* | *4,78* | *4,46* | *11,63* |

### Stade C : Acide (7-chloro-6-nitro-2-oxo-1,2-dihydro-3-quinolinyl)phosphonique diéthyl ester

A une solution de 55 ml d'acide sulfurique 96 % refroidi dans un bain de glace on ajoute goutte à goutte 55 ml d'acide nitrique puis portion par portion l'acide (7-chloro-2-oxo-1,2-dihydro-quinolin-3-yl)-phosphonique diéthyl ester (14,7 g, 46,6 mmol) en maintenant la température inférieure ou égale à 5°C. A la fin de l'addition on poursuit l'agitation 15 minutes puis on retire le bain de glace et la réaction est amenée à température ambiante en 1 heure 30 environ. La solution est versée sur de la glace et le précipité est agité jusqu'à obtenir un solide filtrable. On filtre, lave à l'eau jusqu'à neutralité, sèche sous vide. Le solide est repris en suspension dans 210 ml d'éthanol au reflux, on laisse refroidir et on filtre pour obtenir après séchage le produit du titre.
*Point de fusion : 258-262°C*

| *Microanalyse élémentaire :* | | | | |
|---|---|---|---|---|
| | *C%* | *H%* | *N%* | *Cl%* |
| *calculé* | *43,29* | *3,91* | *7,77* | *9,83* |
| *trouvé* | *43,33* | *4,06* | *7,60* | *9,70* |

### Stade D : Acide (6-amino-7-chloro-2-oxo-1,2-dihydro-3-quinolinyl)phosphonique diéthyl ester

Une suspension du composé obtenu au stade C (7,0 g, 19,4 mmol), de Fer en poudre (10,8 g, 194 mmol), et de chlorure d'ammonium (10,4 g, 194 mmol) est agitée au reflux pendant 1 heure dans un mélange de 270 ml de méthanol et 90 ml d'eau. La suspension est filtrée à chaud sur célite et on rince plusieurs fois le solide avec du méthanol. Le filtrat est évaporé à sec et le résidu est repris en suspension dans de l'eau. On filtre le solide, on rince avec de l'eau et on sèche pour obtenir le produit du titre sous forme de cristaux oranges.
*Point de fusion : 255-260°C*

| *Microanalyse élémentaire :* | | | |
|---|---|---|---|
| | *C%* | *H%* | *N%* |
| *calculé* | *47,22* | *4,88* | *8,47* |
| *trouvé* | *47,06* | *4,99* | *8,08* |

### Stade E : Acide (7-chloro-6-chlorosulfonyl-2-oxo-1,2-dihydro-3-quinolinyl) phosphonique diéthyl ester

Une solution de 13,4 ml d'acide acétique et 2,25 ml d'eau est saturée en SO₂ par barbotage de SO₂ gaz pendant 15 minutes. Parallèlement on prépare à 5°C une solution du composé obtenu au stade D (3,34 g, 10,1 mmol) dans un mélange de 10 ml d'acide acétique glacial et 17 ml d'HCl concentré. A cette solution on ajoute goutte à goutte une solution de nitrite de sodium (767 mg, 11,11 mmol) préalablement dissout dans 5 ml d'eau et on agite la réaction 30 minutes à 5°C. A la solution saturée en SO₂ on rajoute CuCl₂.2H₂O (689 mg, 4,04 mmol) et la suspension obtenue est refroidie à 5°C. A cette dernière on rajoute goutte à goutte la solution du chlorure de diazonium préparée plus haut. Le mélange est agité 1 heure à 5°C puis 3 heures en laissant revenir à température ambiante. La réaction est versée sur de la glace et le précipité est filtré et rincé avec de l'eau. Après séchage on obtient le produit du titre sous forme de poudre jaune pale.
*Point de fusion : 190-200°C*

| *Microanalyse élémentaire :* | | | | | |
|---|---|---|---|---|---|
| | *C%* | *H%* | *N%* | *S%* | *Cl%* |
| *calculé* | *37,70* | *3,41* | *3,38* | *7,74* | *17,12* |
| *trouvé* | *38,04* | *3,47* | *3,40* | *7,74* | *17,16* |

### Stade F : Acide 7-chloro-2-oxo-6-[(n-propylamino)sulfonyl]-1,2-dihydro-3-quinolinyl phosphonique diéthyl ester

On agite pendant 3 heures une suspension du composé obtenu au stade E (1,26 g, 3,0 mmol) avec la n-propylamine (9,0 mmol) dans 30 ml d'acétonitrile. On évapore à sec puis on reprend le résidu dans HCl 1N et on extrait à l'acétate d'éthyle. Après séchage sur MgSO₄ et évaporation de la phase organique, on obtient un résidu qui est repris dans l'éther et filtré pour conduire au produit du titre.
*Point de fusion :* > *300°C*

| *Microanalyse élémentaire :* | | | | |
|---|---|---|---|---|
| | *C%* | *H%* | *N%* | *S%* |
| *calculé* | *43,99* | *5,08* | *6,41* | *7,34* |
| *trouvé* | *43,99* | *5,08* | *6,24* | *7,07* |

### EXEMPLE 2 : Acide 7-chloro-2-oxo-6-[(n-propylamino)sulfonyl]-1,2-dihydro-3-quinolinyl phosphonique

A une suspension du composé obtenu dans l'Exemple 1 (2,53 mmol) dans 30 ml d'acétonitrile anhydre, on ajoute 25,3 mmol de bromotriméthylsilane. On agite à reflux 1 heure et on évapore à sec. Le résidu est séché sous vide et on le reprend dans du méthanol. On agite pendant 30 minutes la suspension qui devient de plus en plus épaisse. Après filtration du précipité et rinçage avec un peu de méthanol, on obtient le produit du titre.
*Point de fusion : > 300°C*

| *Microanalyse élémentaire :* | | | | | |
|---|---|---|---|---|---|
| | *C%* | *H%* | *N%* | *S%* | *Cl%* |
| *calculé* | *37,86* | *3,71* | *7,36* | *8,42* | *9,31* |
| *trouvé* | *37,60* | *3,83* | *7,07* | *7,78* | *10,06* |

Les Exemples suivants sont obtenus selon des modes opératoires semblables à ceux des Exemples 1 et 2 à partir des substrats appropriés.

### EXEMPLE 3 : Acide 7-chloro-6-[(di-n-propylamino)sulfonyl]-2-oxo-1,2-dihydro-3-quinolinyl phosphonique diéthyl ester

On procède comme dans l'Exemple 1 en remplaçant au stade F la n-propylamine par la dipropylamine.
*Point de fusion : 172-175°C*

### EXEMPLE 4 : Acide 7-chloro-6-[(di-n-propylamino)sulfonyl]-2-oxo-1,2-dihydro-3-quinolinyl phosphonique

On procède comme dans l'Exemple 2 à partir du composé obtenu dans l'Exemple 3.
*Point de fusion :* > *300°C*

| *Microanalyse élémentaire :* | | | | | |
|---|---|---|---|---|---|
| | *C%* | *H%* | *N%* | *S%* | *Cl%* |
| *calculé* | *42,61* | *4,77* | *6,63* | *7,58* | *8,38* |
| *trouvé* | *42,40* | *4,78* | *6,48* | *7,35* | *8,95* |

### EXEMPLE 5 : Acide 7-chloro-6-[(méthylamino)sulfonyl]-2-oxo-1,2-dihydro-3-quinolinyl phosphonique diéthyl ester

On procède comme dans l'Exemple 1 en remplaçant au stade F la *n*-propylamine par la méthylamine. La réaction est effectuée dans l'eau. Après acidification avec HCl 3N, on obtient un précipité que l'on filtre qui correspond au produit du titre.
*Point de fusion : 261-265°C*

| Microanalyse élémentaire : | | | | |
|---|---|---|---|---|
| | *C%* | *H%* | *N%* | *S%* |
| *calculé* | *41,13* | *4,44* | *6,85* | *7,84* |
| *trouvé* | *41,05* | *4,39* | *6,83* | *7,86* |

### EXEMPLE 6 : Acide 7-chloro-6-[(méthylamino)sulfonyl]-2-oxo-1,2-dihydro-3-quinolinyl phosphonique

On procède comme dans l'Exemple 2 à partir du composé obtenu dans l'Exemple 5.
*Point de fusion :* > *300°C*

| *Microanalyse élémentaire :* | | | | | |
|---|---|---|---|---|---|
| | *C%* | *H%* | *N%* | *S%* | *Cl%* |
| *calculé* | *34,06* | *2,86* | *7,94* | *9,09* | *10,05* |
| *trouvé* | *34,20* | *3,01* | *7,75* | *8,98* | *10,18* |

### EXEMPLE 7 : Acide 7-chloro-6-[(diméthylamino)sulfonyl]-2-oxo-1,2-dihydro-3-quinolinyl phosphonique diéthyl ester

On procède comme dans l'Exemple 5 en remplaçant au stade F la n-propylamine par la diméthylamine.
*Point de fusion : 237-240°C*

| *Microanalyse élémentaire :* | | | | | |
|---|---|---|---|---|---|
| | *C%* | *H%* | *N%* | *S%* | *Cl%* |
| *calculé* | *42,61* | *4,77* | *6,63* | *7,58* | *8,38* |
| *trouvé* | *42,41* | *4,71* | *6,46* | *7,31* | *8,12* |

### EXEMPLE 8 : Acide 7-chloro-6-[(diméthylamino)sulfonyl]-2-oxo-1,2-dihydro-3-quinolinyl phosphonique

On procède comme dans l'Exemple 2 à partir du composé obtenu dans l'Exemple 7.
*Point de fusion : 256-259°C*

| *Microanalyse élémentaire :* | | | | | |
|---|---|---|---|---|---|
| | *C%* | *H%* | *N%* | *S%* | *Cl%* |
| *calculé* | *34,06* | *2,86* | *7,94* | *9,09* | *10,05* |
| *trouvé* | *34,20* | *3,01* | *7,75* | *8,98* | *10,18* |

### EXEMPLE 9 : Acide 7-chloro-6-(4-morpholinylsulfonyl)-2-oxo-1,2-dihydro-3-quinolinyl phosphonique diéthyl ester

On procède comme dans l'Exemple 1 en remplaçant au stade F la *n*-propylamine par la morpholine.

### EXEMPLE 10 : Acide 7-chloro-6-(4-morpholinylsulfonyl)-2-oxo-1,2-dihydro-3-quinolinyl phosphonique

On procède comme dans l'Exemple 2 à partir du composé obtenu dans l'Exemple 9.
*Point de fusion :* > *260°C*

| *Microanalyse élémentaire :* | | | | | |
|---|---|---|---|---|---|
| | *C%* | *H%* | *N%* | *S%* | *Cl%* |
| *calculé* | *38,20* | *3,45* | *6,85* | *7,84* | *8,67* |
| *trouvé* | *37,90* | *3,64* | *6,55* | *7,59* | *8,70* |

### EXEMPLE 11 : Bromhydrate de l'acide 7-chloro-6-({[2-(diméthylamino)éthyl] amino}sulfonyl)-2-oxo-1,2-dihydro-3-quinolinyl phosphonique diéthyl ester

On procède comme dans l'Exemple 1 en remplaçant au stade F la *n*-propylamine par la diméthylaminoéthylamine. La réaction est réalisée dans la dichlorométhane et après un lavage à l'eau, une purification sur colonne de silice (éluant dichlorométhane/méthanol : 9/1) est réalisée pour conduire au produit du titre.
*Point de fusion : 166-171°C*

| *Microanalyse élémentaire :* | | | | |
|---|---|---|---|---|
| | *C%* | *H%* | *N%* | *S%* |
| *calculé* | *43,83* | *5,41* | *9,02* | *6,88* |
| *trouvé* | *43,50* | *5,48* | *8,70* | *7,05* |

### EXEMPLE 12 : Bromhydrate de l'acide 7-chloro-6-({[2-(diméthylamino)éthyl] amino}sulfonyl)-2-oxo-1,2-dihydro-3-quinolinyl phosphonique

On procède comme dans l'Exemple 2 à partir du composé obtenu dans l'Exemple 11.
*Point de fusion : 216-220°C*

| *Microanalyse élémentaire :* | | | | | |
|---|---|---|---|---|---|
| | *C%* | *H%* | *N%* | *S%* | *Br%* |
| *calculé* | *31,82* | *3,70* | *8,56* | *6,53* | *16,28* |
| *trouvé* | *32,15* | *3,98* | *8,37* | *6,07* | *15,89* |

### EXEMPLE 13 : Acide 6-(anilinosulfonyl)-7-chloro-2-oxo-1,2-dihydro-3-quinolinyl phosphonique diéthyl ester

On procède comme dans l'Exemple 1 en remplaçant au stade F la *n*-propylamine par l'aniline.
*Point de fusion : 139-141°C*

| *Microanalyse élémentaire :* | | | | |
|---|---|---|---|---|
| | *C%* | *H%* | *N%* | *S%* |
| *calculé* | *48,47* | *4,28* | *5,95* | *6,81* |
| *trouvé* | *48,60* | *4,30* | *5,91* | *6,90* |

### EXEMPLE 14 : Acide 6-(anilinosulfonyl)-7-chloro-2-oxo-1,2-dihydro-3-quinolinyl phosphonique

On procède comme dans l'Exemple 2 à partir du composé obtenu dans l'Exemple 13.
*Point de fusion : 249-253°C*

| *Microanalyse élémentaire :* | | | | | |
|---|---|---|---|---|---|
| | *C%* | *H%* | *N%* | *S%* | *Cl%* |
| *calculé* | *43,44* | *2,92* | *6,75* | *7,73* | *8,55* |
| *trouvé* | *43,53* | *2,87* | *6,64* | *7,82* | *9,06* |

### EXEMPLE 15 : Acide 6-[(2-benzoylhydrazino)sulfonyl]-7-chloro-2-oxo-1,2-dihydro-3-quinolinyl phosphonique diéthyl ester

On procède comme dans l'Exemple 1 en remplaçant au stade F la n-propylamine par la benzoylhydrazine. La réaction est réalisée dans le dichlorométhane. Le produit du titre précipite dans le milieu réactionnel et est séparé par filtration.
*Point de fusion : 202-204°C*

| *Microanalyse élémentaire :* | | | | | |
|---|---|---|---|---|---|
| | *C%* | *H%* | *N%* | *S%* | *Cl%* |
| *calculé* | *46,75* | *4,12* | *8,18* | *6,24* | *6,90* |
| *trouvé* | *46,66* | *4,06* | *8,01* | *6,18* | *7,41* |

### EXEMPLE 16 : Acide 6-[(2-benzoylhydrazino)sulfonyl]-7-chloro-2-oxo-1,2-dihydro-3-quinolinyl phosphonique

On procède comme dans l'Exemple 2 à partir du composé obtenu dans l'Exemple 15.
*Point de fusion : 242-244°C*

| *Microanalyse élémentaire :* | | | | | |
|---|---|---|---|---|---|
| | *C%* | *H%* | *N%* | *S%* | *Cl%* |
| *calculé* | *41,98* | *2,86* | *9,18* | *7,00* | *7,74* |
| *trouvé* | *42,12* | *2,80* | *8,95* | *7,05* | *7,92* |

### EXEMPLE 17 : Acide 6-[(2-acétylhydrazino)-sulfonyl]-7-chloro-2-oxo-1,2-dihydro-3-quinolinyl phosphonique diéthyl ester

On procède comme dans l'Exemple 15 en remplaçant au stade F la benzoylhydrazine par l'acétylhydrazine.
*Point de fusion : 125-160°C*

### EXEMPLE 18 : Acide 6-[(2-acétylhydrazino)-sulfonyl]-7-chloro-2-oxo-1,2-dihydro-3-quinolinyl phosphonique

On procède comme dans l'Exemple 2 à partir du composé obtenu dans l'Exemple 17.
*Point de fusion : > 300°C*

| *Microanalyse élémentaire :* | | | | | |
|---|---|---|---|---|---|
| | *C%* | *H%* | *N%* | *S%* | *Cl%* |
| *calculé* | *33,39* | *2,80* | *10,62* | *8,10* | *8,96* |
| *trouvé* | *33,61* | *3,09* | *10,18* | *7,70* | *8,23* |

### EXEMPLE 19 : Acide 7-chloro-6-[(2-{4-[(di-n-propylamino)sulfonyl]benzoyl} hydrazino)sulfonyl]-2-oxo-1,2-dihydro-3-quinolinyl phosphonique diéthyl ester

On procède comme dans l'Exemple 1 en remplaçant au stade F la n-propylamine par la p(dipropylaminosulfonyl)benzoylhydrazine.
*Point de fusion : 288-290°C*

| *Microanalyse élémentaire :* | | | | | |
|---|---|---|---|---|---|
| | *C%* | *H%* | *N%* | *S%* | *Cl%* |
| *calculé* | *46,12* | *5,06* | *8,27* | *9,47* | *5,24* |
| *trouvé* | *45,74* | *5,09* | *8,46* | *9,77* | *5,83* |

### EXEMPLE 20 : Acide 7-chloro-6-[(2-{4-[(di-n-propylamino)sulfonyl]benzoyl} hydrazino)sulfonyl)-2-oxo-1,2-dihydro-3-quinolinyl phosphonique

On procède comme dans l'Exemple 2 à partir du composé obtenu dans l'Exemple 19.
*Point de fusion : 210-214°C*

| *Microanalyse élémentaire :* | | | | | |
|---|---|---|---|---|---|
| | *C%* | *H%* | *N%* | *S%* | *Cl%* |
| *calculé* | *42,55* | *4,22* | *9,02* | *10,33* | *5,71* |
| *trouvé* | *42,35* | *4,45* | *8,69* | *10,52* | *6,21* |

### EXEMPLE 21 : Acide 7-chloro-2-oxo-6-({2-[4-[(trifluorométhyl)benzoyl]hydrazino} sulfonyl)-1,2-dihydro-3-quinolinyl phosphonique diéthyl ester

On procède comme dans l'Exemple 15 en remplaçant au stade F la benzoylhydrazine par la *p*(trifluorométhyl)benzoylhydrazine.
*Point de fusion : 281-282°C*

| *Microanalyse élémentaire :* | | | | |
|---|---|---|---|---|
| | *C%* | *H%* | *N%* | *S%* |
| *calculé* | *43,35* | *3,46* | *7,22* | *5,51* |
| *trouvé* | *43,73* | *3,55* | *7,24* | *5,43* |

### EXEMPLE 22 : Acide 7-chloro-2-oxo-6-({2-[4-[(trifluorométhyl)benzoyl] hydrazino}sulfonyl)-1,2-dihydro-3-quinolinyl phosphonique

On procède comme dans l'Exemple 2 à partir du composé obtenu dans l'Exemple 21.
*Point de fusion : 278-282°C*

| *Microanalyse élémentaire :* | | | | |
|---|---|---|---|---|
| | *C%* | *H%* | *N%* | *S%* |
| *calculé* | *38,83* | *2,30* | *7,99* | *6,10* |
| *trouvé* | *38,80* | *2,26* | *7,78* | *6,01* |

### EXEMPLE 23 : Acide 6-[(benzoylamino)sulfonyl]-7-chloro-2-oxo-1,2-dihydro-3-quinolinyl phosphonique diéthyl ester

On procède comme dans l'Exemple 1 en remplaçant au stade F la *n*-propylamine par la benzoylamine.
*Point de fusion :* > *300°C*

Le composé de l'Exemple 23 peut également être obtenu de la façon suivante :

### Stade A : Acide 6-(aminosulfonyl)-7-chloro-2-oxo-1,2-dihydro -3 -quinolinyl phosphonique diéthyl ester

On agite une suspension du composé obtenu au stade E de l'Exemple 1 (1,26 g, 3,0 mmol) dans 18 ml d'ammoniaque 28 %. Après quelques minutes on observe un passage en solution. On poursuit l'agitation 30 minutes et on acidifie le milieu réactionnel avec HCl 4N. On ajoute sous agitation quelques ml d'acétate d'éthyle et il se produit une précipitation. Le précipité est filtré et séché sous vide pour conduire au produit du titre sous la forme de poudre couleur crème.
*Point de fusion : 288-290°C*

### Stade B : Acide 6-[(benzoylamino)sulfonyl]-7-chloro-2-oxo-1,2-dihydro-3-quinolinyl phosphonique diéthyl ester

Une solution d'acide benzoique (111 mg, 0,912 mmol) dans 3 ml de THF anhydre contenant 173 mg (1,06 mmol) de carbonyldiimidazole est agitée 45 minutes à reflux. Parallèlement on prépare une suspension de 300 mg (0,760 mmol) du composé obtenu au stade A dans 3 ml de THF à laquelle on ajoute 0,125 ml (0,836 mmol) de DBU. On agite 15 minutes à température ambiante puis on y ajoute la solution d'acide benzoique préparée plus haut. On chauffe le mélange 3 heures à 60°C. On refroidit la réaction dans un bain de glace et acidifie par l'addition d'HCl 1N. On extrait à l'acétate d'éthyle. Les phases organiques sont rassemblées et lavées avec une solution saturée de NaCl puis séchées sur MgSO₄. Après évaporation le résidu est chromatographié sur colonne de silice en éluant par un gradient 95/5 90/10 CH₂Cl₂/MeOH. Le produit du titre est obtenu sous la forme d'un solide blanc.

### EXEMPLE 24 : Acide 6-[(benzoylamino)sulfonyl]-7-chloro-2-oxo-1,2-dihydro-3-quinolinyl phosphonique

On procède comme dans l'Exemple 2 à partir du composé obtenu dans l'Exemple 23.
*Point de fusion : 236-241°C*

| *Microanalyse élémentaire :* | | | | | |
|---|---|---|---|---|---|
| | *C%* | *Cl%* | *H%* | *N%* | *S%* |
| *calculé* | *43,40* | *8,01* | *2,73* | *6,33* | *7,24* |
| *trouvé* | *43,38* | *8,18* | *2,86* | *6,17* | *7,20* |

### EXEMPLE 25 : Acide 7-chloro-6-[({4-[(dipropylamino)sulfonyl]benzoyl} amino)sulfonyl]-2-oxo-1,2-dihydro-3-quinolinyl phosphonique diéthyl ester

On procède comme dans l'Exemple 23 en remplaçant l'acide benzoïque par l'acide 4-[(di-n-propylamino)sulfonyl]benzoïque.
*Point de fusion : 197-200°C*

| *Microanalyse élémentaire :* | | | | |
|---|---|---|---|---|
| | *C%* | *H%* | *N%* | *S%* |
| *calculé* | *47,17* | *5,02* | *6,35* | *9,69* |
| *trouvé* | *46,83* | *5,07* | *6,24* | *9,84* |

### EXEMPLE 26 : Acide 7-chloro-6-[({4-[(dipropylamino)sulfonyl]benzoyl} amino)sulfonyl]-2-oxo-1,2-dihydro-3-quinolinyl phosphonique

On procède comme dans l'Exemple 2 à partir du composé obtenu dans l'Exemple 25.
*Point de fusion : 202-206°C*

| *Microanalyse élémentaire :* | | | | | |
|---|---|---|---|---|---|
| | *C%* | *H%* | *N%* | *S%* | *Cl%* |
| *calculé* | *43,60* | *4,16* | *6,93* | *10,58* | *5,85* |
| *trouvé* | *43,81* | *4,27* | *6,72* | *10,62* | *5,96* |

### EXEMPLE 27 : Acide 7-chloro-6-[(méthoxyamino)sulfonyl]-2-oxo-1,2-dihydro-3-quinolinyl phosphonique diéthyl ester

On procède comme dans l'Exemple 1 en remplaçant au stade F la n-propylamine par le chlorhydrate de méthoxyamine et en réalisant la réaction dans un mélange acétonitrile/eau. *Point de fusion : 176-180°C*

| *Microanalyse élémentaire :* | | | | | |
|---|---|---|---|---|---|
| | *C%* | *H%* | *N%* | *S%* | *Cl%* |
| *calculé* | *39,58* | *4,27* | *6,59* | *7,55* | *8,35* |
| *trouvé* | *39,43* | *4,35* | *6,39* | *7,41* | *8,89* |

### EXEMPLE 28 : Acide 7-chloro-6-[(méthoxyamino)sulfonyl]-2-oxo-1,2-dihydro-3-quinolinyl phosphonique

On procède comme dans l'Exemple 2 à partir du composé obtenu dans l'Exemple 27.
*Point de fusion : 259-260°C*

| *Microanalyse élémentaire :* | | | | |
|---|---|---|---|---|
| | *C%* | *H%* | *N%* | *S%* |
| *calculé* | *32,58* | *2,73* | *7,60* | *8,70* |
| *trouvé* | *32,78* | *2,74* | *7,40* | *8,73* |
| | | | | |

### EXEMPLE 29 : Acide 6-[(n-dipropylamino)sulfonyl]-2-oxo-7-(trifluorométhyl)-1,2-dihydro-3-quinolinyl phosphonique diéthyl ester

Les protocoles des stades A-G de l'Exemple 29 sont identiques à ceux de l'Exemple 1 en remplaçant, au stade A de l'Exemple 29 le 2-amino-4-chloro-benzaldéhyde par le 2-amino-4-trifluorométhyl-benzaldéhyde, et en réalisant l'étape de réduction au stade D par le couple Pd-C/formiate d'ammonium au lieu du couple Fe/NH₄Cl en milieu hydroalcoolique.

### Stade A : Acide [(5-trifluorométhyl-2-formyl-phénylcarbamoyl)-méthyl] phosphonique diéthyl ester

*Point de fusion : 62-64°C*

| *Microanalyse élémentaire :* | | | |
|---|---|---|---|
| | *C%* | *H%* | *N%* |
| *calculé* | *45,79* | *4,67* | *3,81* |
| *trouvé* | *45,89* | *4,66* | *3,76* |

### Stade B : Acide (7-trifluorométhyl -2-oxo-1,2-dihydro-3-quinolinyl)phosphonique diéthyl ester

*Point de fusion : 151°C*

| *Microanalyse élémentaire :* | | | |
|---|---|---|---|
| | *C%* | *H%* | *N%* |
| *calculé* | *48,15* | *4,33* | *4,01* |
| *trouvé* | *48,19* | *4,32* | *3,92* |

### Stade C : Acide (7-trifluorométhyl-6-nitro-2 -oxo-1,2-dihydro-3-quinolinyl) phosphonique diéthyl ester

*Point de fusion : 209-215°C*

| *Microanalyse élémentaire :* | | | |
|---|---|---|---|
| | *C%* | *H%* | *N%* |
| *calculé* | *42,65* | *3,58* | *7,11* |
| *trouvé* | *42,86* | *3,58* | *6,78* |

### Stade D : Acide (6-amino-7-trifluorométhyl-2 -oxo-1,2-dihydro-3-quinolinyl) phosphonique diéthyl ester

On agite à reflux pendant 1 heure un mélange de 490 mg (1,24 mmol) du composé obtenu au stade C, 650 mg (12,4 mmol) de formiate d'ammonium et 120 mg de Pd/C 10 % dans 50 ml d'éthanol. Le catalyseur est filtré sur membrane, le filtrat est évaporé à sec et le résidu est repris dans l'eau. On filtre la suspension, rince à l'eau, essore et sèche sous vide pour obtenir le produit du titre sous la forme d'un solide jaune.
*Point de fusion : 240-244°C*

| *Microanalyse élémentaire :* | | | |
|---|---|---|---|
| | *C%* | *H%* | N% |
| *calculé* | *46,16* | *4,43* | *7,69* |
| *trouvé* | *46,26* | *4,37* | *7,62* |

### Stade E : Acide (7-trifluorométhyl-6 -chlorosulfonyl -2-oxo-1,2-dihydro-3-quinolinyl)phosphonique diéthyl ester

*Point de fusion : 165-171°C*

| *Microanalyse élémentaire :* | | | | | |
|---|---|---|---|---|---|
| | *C%* | *H%* | *N%* | *S%* | *Cl%* |
| *calculé* | *34,56* | *3,15* | *3,13* | *7,16* | *7,92* |
| *trouvé* | *37,54* | *3,20* | *3,18* | *7,05* | *7,97* |

### Stade F : Acide 6-[(di-n-propylamino)sulfonyl]-2 -oxo-7-(trifluorométhyl)-1,2-dihydro-3-quinolinyl phosphonique diéthyl ester

On procède comme dans le stade F de l'Exemple 1 en remplaçant la n-propylamine par la dipropylamine.

### EXEMPLE 30 : Acide 6-[(di-n-propylamino)sulfonyl]-2-oxo-7-(trifluorométhyl)-1,2-dihydro-3-quinolinyl phosphonique

On procède comme dans l'Exemple 2 à partir du composé obtenu dans l'Exemple 29.
*Point de fusion :* > *260°C*

| *Microanalyse élémentaire :* | | | | |
|---|---|---|---|---|
| | *C%* | *H%* | *N%* | *S%* |
| *calculé* | *42,11* | *4,42* | *6,14* | *7,03* |
| *trouvé* | *41,87* | *4,19* | *6,03* | *6,79* |

### EXEMPLE 31: Acide 2-oxo-6-(1-pipéridinylsulfonyl)-7-(trifluorométhyl)-1,2-dihydro-3-quinolinyl phosphonique diéthyl ester

On procède comme dans l'Exemple 29 en remplaçant au stade F la dipropylamine par la pipéridine.
*Point de fusion : 132-135°C*

### EXEMPLE 32: Acide 2-oxo-6-(1-pipéridinylsulfonyl)-7-(trifluorométhyl)-1,2-dihydro-3-quinolinyl phosphonique

*Point de fusion : > 260°C*

| *Microanalyse élémentaire :* | | | | |
|---|---|---|---|---|
| | *C%* | *H%* | *N%* | *S%* |
| *calculé* | *40,92* | *3,66* | *6,36* | *7,28* |
| *trouvé* | *40,96* | *3,64* | *6,31* | *7,25* |

### EXEMPLE 33 : Acide 6-{[(benzyloxy)amino]sulfonyl}-7-chloro-2-oxo-1,2-dihydro-3-quinolinyl phosphonique diéthyl ester

On procède comme dans l'exemple 27 en remplaçant le chlorhydrate de méthoxyamine par le chlorhydrate de benzyloxyamine.
*Point de fusion : 233-236°C*

| *Microanalyse élémentaire :* | | | | |
|---|---|---|---|---|
| | *C%* | *H%* | *N%* | *S%* |
| *calculé* | *47,96* | *4,43* | *5,59* | *6,40* |
| *trouvé* | *47,84* | *4,83* | *5,56* | *6,24* |

### EXEMPLE 34 : Acide 7-chloro-6-[(hydroxyamino)sulfonyl]-2-oxo-1,2-dihydro-3-quinolinyl phosphonique diéthyl ester

A une suspension de 310 mg (0,655 mmol) du produit du stade précédent dans 30 ml de CH₂Cl₂ refroidit à -60°C on additionne goutte à goutte 1,35 ml (1,35 mmol) d'une solution de BBr₃ 1M dans le CH₂Cl_{2.} On laisse revenir le milieu réactionnel à -20 °C en 1 heure environ et on verse sur de la glace. On agite la suspension blanche gélatineuse puis on la filtre. Le solide blanc est rinçé plusieurs fois avec de l'eau puis de l'éther. On le remet en suspension à chaud dans l'acétonitrile et filtre le solide obtenu correspondant au produit du titre.
*Point de fusion : 285-290°C*

| *Microanalyse élémentaire :* | | | | |
|---|---|---|---|---|
| | *C%* | *H%* | *N%* | *S%* |
| *calculé* | *38,01* | *3,93* | *6,82* | *7,81* |
| *trouvé* | *38,01* | *3,97* | *6,48* | *7,86* |

### EXEMPLE 35 : Acide 7-chloro-6-[(hydroxyamino)sulfonyl]-2-oxo-1,2-dihydro-3-quinolinyl phosphonique

On procède comme dans l'Exemple 2 à partir du composé obtenu dans l'Exemple 34.
*Point de fusion : 282-290°C*

| *Microanalyse élémentaire :* | | | | | |
|---|---|---|---|---|---|
| | *C%* | *H%* | *N%* | *S%* | *Cl%* |
| *calculé* | *30,48* | *2,27* | *7,90* | *9,04* | *10,00* |
| *trouvé* | *30,76* | *2,58* | *7,35* | *9,03* | *10,04* |

### EXEMPLE 36 : Acide 7-chlore-6-[(cyclopropylamino)sulfonyl]-2-oxo-1,2-dihydro-3-quinolinyl phosphonique diéthyl ester

On procède comme dans l'exemple 1 en remplaçant au stade F la *n*-propylamine par la cyclopropylamine.
*Point de fusion : 261-263°C*

| *Microanalyse élémentaire :* | | | | | |
|---|---|---|---|---|---|
| | *C%* | *H%* | *N%* | *S%* | *Cl%* |
| *calculé* | *44,20* | *4,64* | *6,44* | *7,37* | *8,15* |
| *trouvé* | *44,13* | *4,75* | *6,03* | *6,82* | *8,70* |

### EXEMPLE 37 : Acide 7-chloro-6-[(cyclopropylamino)sulfonyl]-2-oxo-1,2-dihydro-3-quinolinyl phosphonique

On procède comme dans l'Exemple 2 à partir du composé obtenu dans l'Exemple 36.
*Point de fusion : > 300°C*

| *Microanalyse élémentaire :* | | | | | |
|---|---|---|---|---|---|
| | *C%* | *H%* | *N%* | *S%* | *Cl%* |
| *calculé* | *38,06* | *3,19* | *7,40* | *8,47* | *9,36* |
| *trouvé* | *38,05* | *3,18* | *7,08* | *8,27* | *10,47* |

### EXEMPLE 38 : Acide 7-chloro-6-[(isopropylamino)sulfonyl]-2-oxo-1,2-dihydro-3-quinolinyl phosphonique diéthyl ester

On procède comme dans l'exemple 1 en remplaçant au stade F la n-propylamine par l'isopropylamine.
*Point de fusion : 239-240°C*

| *Microanalyse élémentaire :* | | | | | |
|---|---|---|---|---|---|
| | *C%* | *H%* | *N%* | *S%* | *Cl%* |
| *calculé* | *43,99* | *5,08* | *6,41* | *7,34* | *8,12* |
| *trouvé* | *44,13* | *5,13* | *6,24* | *7,25* | *9,76* |

### EXEMPLE 39 : Acide 7-chloro-6-[(isopropylamino)sulfonyl]-2-oxo-1,2-dihydro-3-quinolinyl phosphonique

On procède comme dans l'Exemple 2 à partir du composé obtenu dans l'Exemple 38.
*Point de fusion : > 300°C*

| Microanalyse élémentaire : | | | | | |
|---|---|---|---|---|---|
| | *C%* | *H%* | *N%* | *S%* | *Cl%* |
| *calculé* | *37,86* | *3.71* | *7,36* | *8,42* | *9,31* |
| *trouvé* | *37,56* | *3,83* | *7,09* | *7,98* | *9,85* |

### EXEMPLE 40 : Acide 6-[(n-butylamino)sulfonyl]-7-chloro-2-oxo-1,2-dihydro-3-quinolinyl phosphonique diéthyl ester

On procède comme dans l'exemple 1 en remplaçant au stade F la *n*-propylamine par la *n*-butylamine.
*Point de fusion : 229-230°C*

### EXEMPLE 41 : Acide 6-[(n-butylamino)sulfonyl]-7-chloro-2-oxo-1,2-dihydro-3-quinolinyl phosphonique

On procède comme dans l'Exemple 2 à partir du composé obtenu dans l'Exemple 40.
*Point de fusion :* > *300°C*

| *Microanalyse élémentaire :* | | | | | |
|---|---|---|---|---|---|
| | *C%* | *H%* | *N%* | *S%* | *Cl%* |
| *calculé* | *39,55* | *4,08* | *7,10* | 8,12 | *8,98* |
| *trouvé* | *39,25* | *4,05* | *6,86* | *7,63* | *9,77* |

### EXEMPLE 42 : Acide 7-chloro-6-[(éthylamino)sulfonyl]-2-oxo-1,2-dihydro-3-quinolinyl phosphonique diéthyl ester

On procède comme dans l'exemple 1 en remplaçant au stade F la n-propylamine par l'éthylamine.
*Point de fusion : 225°C*

### EXEMPLE 43 : Acide 7-chloro-6-[(éthylamino)sulfonyl]-2-oxo-1,2-dihydro-3-quinolinyl phosphonique

On procède comme dans l'Exemple 2 à partir du composé obtenu dans l'Exemple 42.
*Point de fusion :* > *300°C*

| *Microanalyse élémentaire :* | | | | | |
|---|---|---|---|---|---|
| | *C%* | *H%* | *N%* | *S%* | *Cl%* |
| *calculé* | *36,03* | *3,30* | *7,64* | *8,74* | *9,67* |
| *trouvé* | *35,95* | *3,44* | *7,31* | *8,16* | *10,51* |

### EXEMPLE 44 : Acide 7-chloro-2-oxo-6-{[(2-phényléthyl)amino]sulfonyl}-1,2-dihydro-3-quinolinyl phosphonique diéthyl ester

On procède comme dans l'exemple 1 en remplaçant au stade F la *n*-propylamine par la phénéthylamine.
*Point de fusion : 267-268°C*

| *Microanalyse élémentaire :* | | | | | |
|---|---|---|---|---|---|
| | *C%* | *H%* | *N%* | *S%* | *Cl%* |
| *calculé* | *50,56* | *4,85* | *5,61* | *6,43* | *7,11* |
| *trouvé* | *50,71* | *4,94* | *5,68* | *6,22* | *7,92* |

### EXEMPLE 45 : Acide 7-chloro-2-oxo-6-{[(2-phényléthyl)amino]sulfonyl}-1,2-dihydro-3-quinolinyl phosphonique

On procède comme dans l'Exemple 2 à partir du composé obtenu dans l'Exemple 44.
*Point de fusion :* > *300°C*

| *Microanalyse élémentaire :* | | | | | |
|---|---|---|---|---|---|
| | *C%* | *H%* | *N%* | *S%* | *Cl%* |
| *calculé* | *46,11* | *3,64* | *6,33* | *7,24* | *8,01* |
| *trouvé* | *45,60* | *3,65* | *6,18* | *7,06* | *8,53* |

### EXEMPLE 46 : Acide 2-oxo-6-[(n-propylamino)sulfonyl]-7-(trifluorométhyl)-1,2-dihydro-3-quinolinyl phosphonique diéthyl ester

On procède comme au stade F de l'Exemple 29 en remplaçant la dipropylamine par la *n*-propylamine.
*Point de fusion : 218-220°C*

| *Microanalyse élémentaire :* | | | | |
|---|---|---|---|---|
| | *C%* | *H%* | *N%* | *S%* |
| *calculé* | *43,41* | *4,71* | *5,96* | *6,82* |
| *trouvé* | *43,39* | *4,65* | *5,82* | *6,80* |

### EXEMPLE 47 : Acide 2-oxo-6-[(n-propylamino)sulfonyl]-7-(trifluorométhyl)-1,2-dihydro-3-quinolinyl phosphonique

On procède comme dans l'Exemple 2 à partir du composé obtenu dans l'Exemple 46.
*Point de fusion : 277°C*

| *Microanalyse élémentaire :* | | | | |
|---|---|---|---|---|
| | *C%* | *H%* | *N%* | *S%* |
| *calculé* | *37,69* | *3,41* | *6,76* | *7,74* |
| *trouvé* | *37,74* | *3,57* | *6,74* | *8,10* |

### EXEMPLE 48 : Acide 6-[(méthylamino)sulfonyl]-2-oxo-7-(trifluorométhyl)-1,2-dihydro-3-quinolinyl phosphonique diéthyl ester

On procède comme au stade F de l'exemple 29 en remplaçant la dipropylamine par la méthylamine.
*Point de fusion : 142-145°C*

| *Microanalyse élémentaire :* | | | | |
|---|---|---|---|---|
| | *C%* | *H%* | *N%* | *S%* |
| *calculé* | *40,73* | *4,10* | *6,33* | *7,25* |
| *trouvé* | *40,64* | *4,01* | *6,18* | *7,11* |

### EXEMPLE 49 : Acide 6-[(méthylamino)sulfonyl]-2-oxo-7-(trifluorométhyl)-1,2-dihydro-3-quinolinyl phosphonique

On procède comme dans l'Exemple 2 à partir du composé obtenu dans l'Exemple 48.
*Point de fusion : > 300°C*

| *Microanalyse élémentaire :* | | | | |
|---|---|---|---|---|
| | *C%* | *H%* | *N%* | *S%* |
| *calculé* | *34,21* | *2,61* | *7,25* | *8,30* |
| *trouvé* | *34,56* | *2,66* | *7,34* | *8,59* |

### ETUDE PHARMACOLOGIQUE

### EXEMPLE A : Inhibition du courant induit par une application de (R,S)-AMPA (10 µM) sur des ovocytes de Xenope injectés avec des ARNm de cortex de rat

Des ovocytes de Xenopes sont injectés avec 50 ng d'ARNm poly (A+) isolés de cortex cérébral de rat et incubés 2 à 3 jours à 18°C pour en permettre l'expression protéique. Les courants entrants induits par une application de (R,S)-AMPA (10 µM) sont mesurés dans un milieu de composition : NaCl (82,5 mM), KCl ( 2,5 mM), CaCl₂ (1 mM), MgCl₂ (1 mM), NaH₂PO₄ (1 mM), HEPES (5 mM), pH 7,4 par la méthode du voltage-clamp à 2 électrodes (potentiel = - 60 mV). Les produits de la présente invention sont appliqués de façon concentration dépendante 30 secondes avant et durant l'application de l'agoniste (R,S)-AMPA.
Leur capacité à inhiber le courant induit par le (R,S)-AMPA est déterminée par les valeurs d'IC50 (µM), représentant les concentrations inhibitant de 50 % le courant induit par une application de (R,S)-AMPA (10 µM).
Les composés de l'invention montrent d'excellentes propriétés inhibitrices avec des IC50 (µM) de l'ordre de 1.

### EXEMPLE B: Test des convulsions audiogènes chez la souris DBA/2

Chez la souris DBA/2 immature, des crises convulsives peuvent être déclenchées en soumettant cet animal à une stimulation sonore de haute intensité et de haute fréquence.
Les antagonistes des récepteurs au glutamate de type AMPA antagonisent ce type de convulsions de façon dose-dépendante (Chapman et al., Epilepsy Res., 1991, 9, 92-96). Ce test est utilisé pour étudier les effets anti-convulsivants des composés de la présente invention. Brièvement, des souris DBA/2 immatures (21-28 jours) sont exposées pendant 60 secondes à un bruit de 105 Db et de 18 KH_{z}. Ceci entraîne l'apparition de convulsions cloniques. Les produits sous étude et le solvant sont administrés par voie i.p. 30 minutes avant le début du test sous un volume de 0,1 ml/10 g. Les ED50 (doses inhibant de 50 % l'incidence des convulsions) est déterminée pour chaque composé en utilisant la méthode de Litchfield et Wicoxon (J. Pharmacol. Exp. Ther., 1949, 96, 99-113).
Les composés de l'invention montrent une excellente capacité à inhiber les convulsions avec des ED50 de l'ordre de 10 mg/kg ip.

### EXEMPLE C : Test de néphrotoxicité chez le rat Fischer

L'évaluation de l'impact rénal des composés de la présente invention est réalisée chez le rat adulte mâle Fischer de 200-250 g. Des rats Fischer sont anesthésiés au pentobarbital (Nembutal®, 60 mg/kg i.p.). Quatre-vingt-dix minutes après l'induction de l'anesthésie, les composés sous étude sont administrés par voie intraveineuse aux doses de 3 et 10 mg/kg. Vingt-quatre heures après administration, les animaux sont sacrifiés, le plasma est prélevé et la créatinémie et l'urémie sont mesurées. L'analyse statistique est réalisée à l'aide d'une analyse de variance à un facteur suivie d'un test de Newman-Keuls, en comparant les animaux traités et les animaux ayant uniquement reçu le véhicule.
Les composés de l'invention présentent une excellente tolérance rénale, aucun effet toxique n'étant obtenu pour des doses inférieures ou égales à 10 mg/kg iv.

### EXEMPLE D : Composition pharmaceutique

| | |
|---|---|
| 1000 comprimés dosés à 5 mg de l'acide 7-chloro-2-oxo-6-[(*n*-propylamino)sulfonyl]-1,2-dihydro-3-quinolinyl phosphonique (Exemple 2) | 5 g |
| Amidon de blé | 20 g |
| Amidon de maïs | 20 g |
| Lactose | 30 g |
| Stéarate de magnésium | 2 g |
| Silice | 1 g |
| Hydroxypropylcellulose | 2 g |

## Revendications

1. Composés de formule (I) : 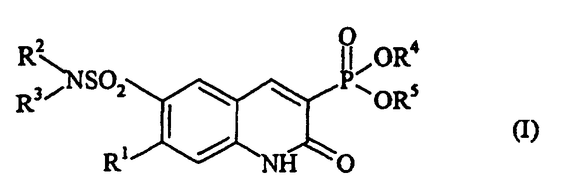 dans laquelle :
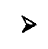 R¹ représente un atome d'halogène ou un groupement trifluorométhyle,
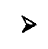 R² représente un atome d'hydrogène ou un groupement alkyle ou cycloalkyle,
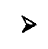 R³ représente un groupement alkyle, cycloalkyle, aryle, arylalkyle, alkoxy, aryloxy, arylalkoxy, hydroxy, aminoalkyle, alkylaminoalkyle, dialkylaminoalkyle, alkylcarbonyle, arylcarbonyle, arylalkylcarbonyle, ou NHR⁶ (où R⁶ représente un atome d'hydrogène ou un groupement alkyle, cycloalkyle, aryle, alkylcarbonyle, arylcarbonyle, arylalkyle ou arylalkylcarbonyle),
ou R² et R³ forment ensemble, avec l'atome d'azote qui les porte un cycle à 5 ou 6 atomes de carbone dans lequel un des atomes de carbone peut être remplacé par un atome d'oxygène, d'azote ou de soufre ou par un groupement SO, SO₂ ou NRₐ (dans lequel Rₐ représente un atome d'hydrogène ou un groupement alkyle, cycloalkyle, aryle ou arylalkyle),
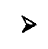 R⁴ et R⁵, identiques ou différents, représentent un atome d'hydrogène ou un groupement alkyle, cycloalkyle, aryle, arylalkyle ou un groupement 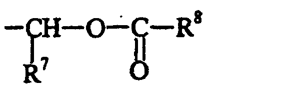 (dans lequel R⁷ et R⁸, identiques ou différents, représentent un atome d'hydrogène ou un groupement alkyle, cycloalkyle ou aryle),
étant entendu que :
- par alkyle on entend un groupement alkyle contenant 1 à 6 atomes de carbone, linéaire ou ramifié,
- par alkoxy on entend un groupement alkoxy contenant 1 à 6 atomes de carbone, linéaire ou ramifié,
- par cycloalkyle on entend un groupement alkyle cyclique contenant de 3 à 8 atomes de carbone,
- par aryle on entend les groupements phényle ou naphtyle, ces groupements pouvant être non substitués ou substitués par 1 à 3 groupements choisis parmi alkyle, cycloalkyle, hydroxy, alkoxy, amino, alkylamino, dialkylamino, cyano, nitro, polyhalogénoalkyle, SO₂NR⁹R¹⁰ (où R⁹ et R¹⁰, identiques ou différents, représentent un atome d'hydrogène ou un groupement alkyle, cycloalkyle ou aryle), ou atomes d'halogène,
leurs énantiomères et diastéréoisomères ainsi que leurs sels d'addition à un acide ou à une base pharmaceutiquement acceptable.

2. Composés de formule (I) selon la revendication 1 pour lesquels R¹ représente un atome de chlore, leurs isomères ainsi que leurs sels d'addition à un acide ou à une base pharmaceutiquement acceptables.

3. Composés de formule (I) selon la revendication 1 pour lesquels R³ représente un groupement alkyle, leurs isomères ainsi que leurs sels d'addition à un acide ou à une base pharmaceutiquement acceptable.

4. Composés de formule (I) selon la revendication 1 pour lesquels R³ représente un groupement aryle, leurs isomères ainsi que leurs sels d'addition à un acide ou à une base pharmaceutiquement acceptables.

5. Composés de formule (I) selon la revendication 1 pour lesquels R³ représente un groupement NHCOR dans lequel R représente un groupement alkyle ou aryle, leurs isomères ainsi que leurs sels d'addition à un acide ou à une base pharmaceutiquement acceptables.

6. Composés de formule (I) selon la revendication 1 pour lesquels R² représente un atome d'hydrogène, leurs isomères ainsi que leurs sels d'addition à un acide ou à une base pharmaceutiquement acceptables.

7. Composés de formule (I) selon la revendication 1 pour lesquels R⁴ et R⁵ représentent simultanément un atome d'hydrogène, leurs isomères ainsi que leurs sels d'addition à un acide ou à une base pharmaceutiquement acceptables.

8. Composés de formule (I) selon la revendication 1 qui sont l'acide 7-chloro-2-oxo-6-[(propylamino)sulfonyl]-1,2-dihydro-3-quinolinyl phosphonique, et l'acide 7-chloro-6-[(méthylamino)sulfonyl]-2-oxo-1,2-dihydro-3-quinolinyl phosphonique, leurs isomères ainsi que leurs sels d'addition à un acide ou à une base pharmaceutiquement acceptables.

9. Composé de formule (I) selon la revendication 1 qui est l'acide 7-chloro-6-[(2-{4-[(dipropylamino)sulfonyl]benzoyl}hydrazino)sulfonyl]-2-oxo-1,2-dihydro-3-quinolinyl phosphonique, ses isomères ainsi que ses sels d'addition à un acide ou à une base pharmaceutiquement acceptables.

10. Composé de formule (I) selon la revendication 1 qui est le bromhydrate de l'acide 7-chloro-6-({[2-(diméthylamino)éthyl]amino}sulfonyl)-2-oxo-1,2-dihydro-3-quinolinyl phosphonique, ses isomères ainsi que ses sels d'addition à un acide ou à une base pharmaceutiquement acceptables.

11. Composé de formule (I) selon la revendication 1 qui est l'acide 6-(anilinosulfonyl)-7-chloro-2-oxo-1,2-dihydro-3-quinolinyl phosphonique, ses isomères ainsi que ses sels d'addition à un acide ou à une base pharmaceutiquement acceptables.

12. Composé de formule (I) selon la revendication 1 qui est l'acide 6-[(2-benzoylhydrazino)sulfonyl]-7-chloro-2-oxo-1,2-dihydro-3-quinolinyl phosphonique, ses isomères ainsi que ses sels d'addition à un acide ou à une base pharmaceutiquement acceptables.

13. Procédé de préparation des composés de formule (I) selon la revendication 1 **caractérisé en ce que** l'on utilise comme produit de départ le composé de formule (II) : 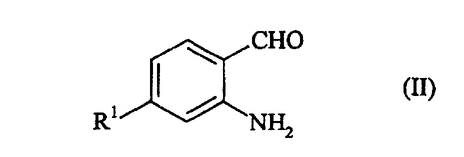 dans laquelle R¹ est tel que défini dans la formule (I),
sur lequel on condense en présence de base comme la pyridine par exemple un composé de formule (III) : 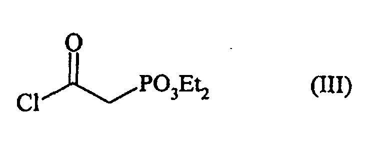 pour conduire au composé de formule (IV) 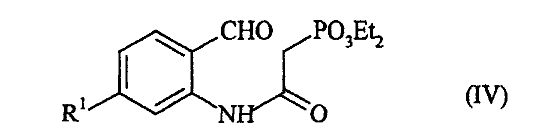 dans laquelle R¹ est tel que défini précédemment,
que l'on cyclise en présence d'une quantité catalytique de pipéridine pour obtenir le composé de formule (V) : 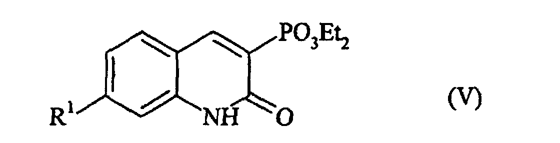 dans laquelle R¹ est défini comme précédemment,
que l'on soumet à un mélange d'acide nitrique et d'acide sulfurique pour conduire au composé de formule (VI) : 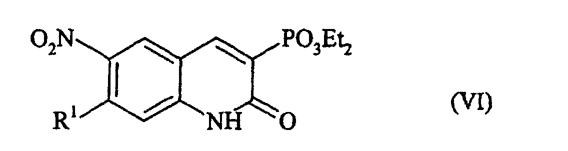 dans laquelle R¹ est tel que défini précédemment,
qui est réduit en utilisant du charbon palladié en présence d'hydrogène ou du Fer en milieu hydroalcoolique pour conduire au composé de formule (VII) : 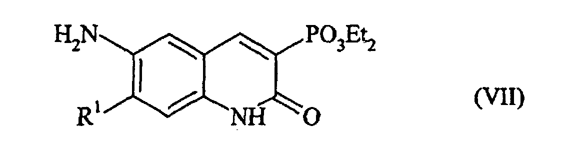 dans laquelle R¹ est défini comme précédemment,
qui est soumis, après transformation en sel de diazonium correspondant, à l'action de dioxide de soufre en présence de CuCl₂ pour conduire au composé de formule (VIII) : 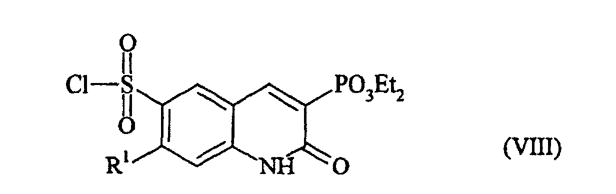 dans laquelle R¹ est tel que défini précédemment,
sur lequel on condense un composé de formule HNR²R³ dans lequel R² et R³ sont tels que définis dans la formule (I) pour conduire au composé de formule (I/a), cas particulier des composés de formule (I) : 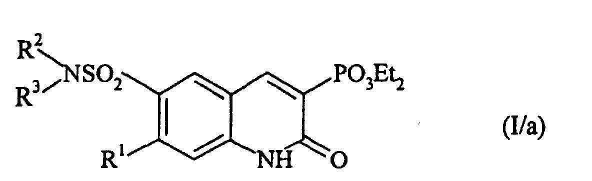 dans laquelle R¹, R² et R³ sont tels que définis précédemment,
qui est partiellement ou totalement déprotégé en présence de bromure de triméthylsilane par exemple pour conduire au composé de formule (I/b), cas particulier des composés de formule (I) : 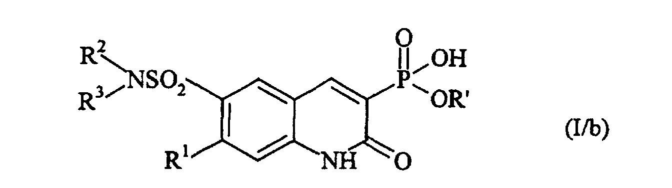 dans laquelle R¹, R² et R³ sont tels que définis précédemment et R' représente un atome d'hydrogène ou un groupement éthyle,
sur lequel on peut condenser un composé de formule (IX) :
R"-Cl (IX)
dans laquelle R" représente un groupement alkyle, aryle, arylalkyle ou 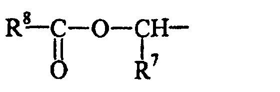 (où R⁷ et R⁸ sont définis comme dans la formule (I)),
pour obtenir le composé de formule (I/c), cas particulier des composés de formule (I) : 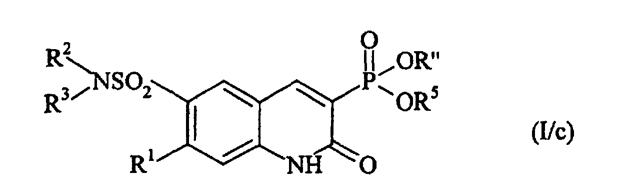 dans laquelle R¹, R², R³, R⁵ et R" sont tels que définis précédemment,
les composés de formule (I/a) à (I/c) formant l'ensemble des composés de formule (I), et pouvant être purifiés selon une technique classique de séparation, que l'on transforme, si on le souhaite en leurs sels d'addition à un acide ou à une base pharmaceutiquement acceptable et dont on sépare éventuellement les isomères selon une technique classique de séparation.

14. Compositions pharmaceutiques contenant comme principe actif au moins un composé de formule (I) selon l'une quelconque des revendications 1 à 12 ou un de ses sels d'addition à un acide ou à une base pharmaceutiquement acceptables, seul ou en combinaison avec un ou plusieurs excipients pharmaceutiquement acceptables.

15. Compositions pharmaceutiques selon la revendication 14 utiles pour la fabrication d'un médicament pour le traitement aigu et chronique des phénomènes pathologiques liés à l'hyperactivation des voies de neurotransmission aux aminoacides excitateurs comme l'accident vasculaire cérébral, le traumatisme cérébral ou spinal, l'épilepsie, les maladies neurodégénératives chroniques telles que la maladie d'Alzheimer, la schizophrénie, la sclérose amyotrophique latérale ou la chorée de Huntington

## Patentansprüche

1. Verbindungen der Formel (I): 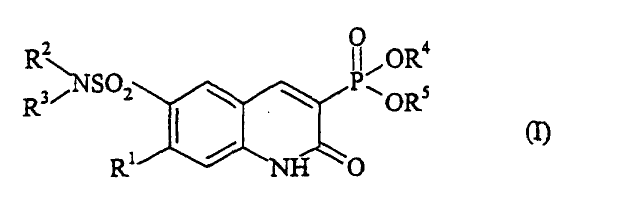 in der:
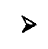 R¹ ein Halogenatom oder eine Trifluormethylgruppe darstellt,
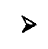 R² ein Wasserstoffatom oder eine Alkyl- oder Cycloalkylgruppe darstellt,
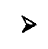 R³ eine Alkyl-, Cycloalkyl-, Aryl-, Arylalkyl-, Alkoxy-, Aryloxy-, Arylalkoxy-, Hydroxy-, Aminoalkyl-, Alkylaminoalkyl-, Dialkylaminoalkyl-, Alkylcarbonyl-, Arylcarbonyl-, Arylalkylcarbonylgruppe oder eine Gruppe NHR⁶ (worin R⁶ ein Wasserstoffatom oder eine Alkyl-, Cycloalkyl-, Aryl-, Alkylcarbonyl-, Arylcarbonyl-, Arylalkyl- oder Arylalkylcarbonylgruppe darstellt) bedeutet,
oder R² und R³ gemeinsam mit dem sie tragenden Stickstoffatom einen Ring mit 5 oder 6 Kohlenstoffatomen bilden, in dem eines der Kohlenstoffatome durch ein Sauerstoff-, Stickstoff- oder Schwefelatom oder eine Gruppe SO, SO₂ oder NRₐ (worin Rₐ ein Wasserstoffatom oder eine Alkyl-, Cycloalkyl-, Aryl- oder Arylalkylgruppe darstellt) ersetzt sein kann,
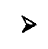 R⁴ und R⁵, die gleichartig oder verschieden sind, Wasserstoffatome oder Alkyl-, Cycloalkyl-, Aryl-, Arylalkylgruppne oder Gruppen 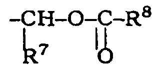 (worin R⁷ und R⁸, die gleichartig oder verschieden sind, Wasserstoffatome oder Alkyl-, Cycloalkyl- oder Arylgruppen darstellen) bedeuten,
mit der Maßgabe, daß:
- man unter Alkyl eine geradkettige oder verzweigte Alkylgruppe versteht, die 1 bis 6 Kohlenstoffatome enthält,
- man unter Alkoxy eine geradkettige oder verzweigte Alkoxygruppe versteht, die 1 bis 6 Kohlenstoffatome enthält,
- man unter Cycloalkyl eine cyclische Alkylgruppe versteht, die 3 bis 8 Kohlenstoffatome enthält,
- man unter Aryl Phenyl- oder Naphthylgruppen versteht, wobei diese Gruppen nichtsubstituiert oder durch 1 bis 3 Gruppen substituiert sein können ausgewählt aus Alkyl, Cycloalkyl, Hydroxy, Alkoxy, Amino, Alkylamino, Dialkylamino, Cyano, Nitro, Polyhalogenalkyl, SO₂NR⁹R¹⁰ (worin R⁹ und R¹⁰, die gleichartig oder verschieden sind, Wasserstoffatome oder Alkyl-, Cycloalkyl- oder Arylgruppen bedeuten) oder Halogenatomen,
deren Enantiomere und Diastereoisomere sowie deren Additionssalze mit einer pharmazeutisch annehmbaren Säure oder Base.

2. Verbindungen der Formel (I) nach Anspruch 1, worin R¹ ein Chloratom darstellt, deren Isomere sowie deren Additionssalze mit einer pharmazeutisch annehmbaren Säure oder Base.

3. Verbindungen der Formel (I) nach Anspruch 1, worin R³ eine Alkylgruppe darstellt, deren Isomere sowie deren Additionssalze mit einer pharmazeutisch annehmbaren Säure oder Base.

4. Verbindungen der Formel (I) nach Anspruch 1, worin R³ eine Arylgruppe darstellt, deren Isomere sowie deren Additionssalze mit einer pharmazeutisch annehmbaren Säure oder Base.

5. Verbindungen der Formel (I) nach Anspruch 1, worin R³ eine Gruppe NHCOR darstellt, worin R eine Alkyl- oder Arylgruppe bedeutet, deren Isomere sowie deren Additionssalze mit einer pharmazeutisch annehmbaren Säure oder Base.

6. Verbindungen der Formel (I) nach Anspruch 1, worin R² ein Wasserstoffatom darstellt, deren Isomere sowie deren Additionssalze mit einer pharmazeutisch annehmbaren Säure oder Base.

7. Verbindungen der Formel (I) nach Anspruch 1, worin R⁴ und R⁵ gleichzeitig Wasserstoffatome bedeuten, deren Isomere sowie deren Additionssalze mit einer pharmazeutisch annehmbaren Säure oder Base.

8. Verbindungen der Formel (I) nach Anspruch 1, nämlich 7-Chlor-2-oxo-6-[(propylamino)-sulfonyl]-1,2-dihydro-3-chinolinyl-phosphonsäure und 7-Chlor-6-[(methylamino]-sulfonyl]-2-oxo-1,2-dihydro-3-chinolinyl-phosphonsäure, deren Isomere sowie deren Additionssalze mit einer pharmazeutisch annehmbaren Säure oder Base.

9. Verbindung der Formel (I) nach Anspruch 1, nämlich 7-Chlor-6-[(2-{4-[(dipropylamino)-sulfonyl]-benzoyl}-hydrazino)-sulfonyl]-2-oxo-1,2-dihydro-3-chinolinyl-phosphonsäure, deren Isomere sowie deren Additionssalze mit einer pharmazeutisch annehmbaren Säure oder Base.

10. Verbindung der Formel (I) nach Anspruch 1, nämlich 7-Chlor-6-({[2-(Dimethylamino)-ethyl]-amino}-sulfonyl)-2-oxo-1,2-dihydro-3-chinolinyl-phophonsäure-Hydrobromid, deren Isomere sowie deren Additionssalze mit einer pharmazeutisch annehmbaren Säure oder Base.

11. Verbindung der Formel (I) nach Anspruch 1, nämlich 6-(Anilinosulfonyl)-7-chlor-2-oxo-1,2-dihydro-3-chinolinyl-phosphonsäure, deren Isomere sowie deren Additionssalze mit einer pharmazeutisch annehmbaren Säure oder Base.

12. Verbindung der Formel (I) nach Anspruch 1, nämlich 6-[(2-Benzoylhydrazino)-sulfonyl]-7-chlor-2-oxo-1,2-dihydro-3-chinolinyl-phosphonsäure, deren Isomere sowie deren Additionssalze mit einer pharmazeutisch annehmbaren Säure oder Base.

13. Verfahren zur Herstellung de Verbindungen der Formel (I) nach Anspruch 1, **dadurch gekennzeichnet, daß** man als Ausgangsprodukt die Verbindung der Formel (II) verwendet: 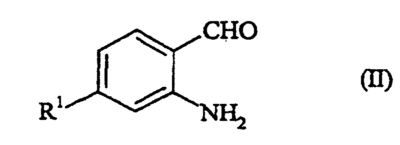 in der R¹ die bezüglich der Formel (I) angegebenen Bedeutungen besitzt, welche man in Gegenwart einer Base, wie beispielsweise Pyridin, mit einer Verbindung der Formel (III) kondensiert: 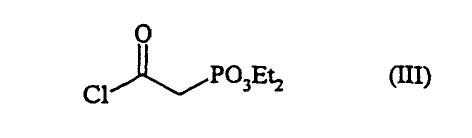 zur Bildung der Verbindung der Formel (IV): 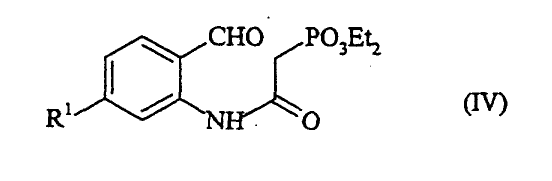 in der R¹ die oben angegebenen Bedeutungen besitzt,
welche man in Gegenwart einer katalytischen Menge Piperidins cyclisiert zur Bildung der Verbindung der Formel (V): 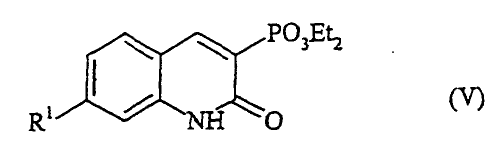 in der R¹ die oben angegebenen Bedeutungen besitzt,
welche man mit einer Mischung aus Salpetersäure und Schwefelsäure behandelt zur Bildung der Verbindung der Formel (VI): 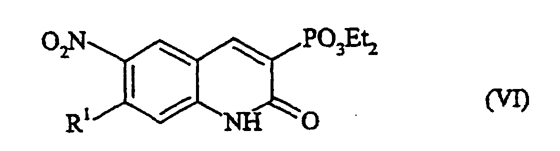 in der R¹ die oben angegebenen Bedeutungen besitzt,
welche man unter Verwendung von Palladium-auf-Kohlenstoff in Gegenwart von-Wasserstoff oder von Eisen in wäßrigen-alkoholischem Medium reduziert zur Bildung der Verbindung der Formel (VII): 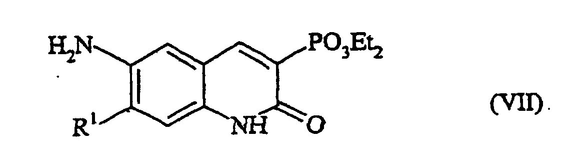 in der R¹ die oben angegebenen Bedeutungen besitzt,
welche man nach der Umwandlung in das entsprechende Diazoniumsalz der Einwirkung von Schwefeldioxid in Gegenwart von CuCl₂ unterwirft zur Bildung der Verbindung der Formel (VIII): 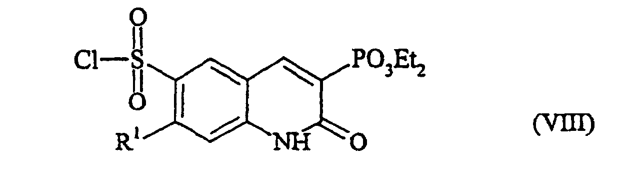 in der R¹ die oben angegebenen Bedeutungen besitzt,
welche man mit einer Verbindung der Formel HNR²R³, in der R² und R³ die bezüglich der Formel (I) angegebenen Bedeutungen besitzen, kondensiert zur Bildung der Verbindung der Formel (I/a). einem Sonderfall der Verbindungen der Formel (I): 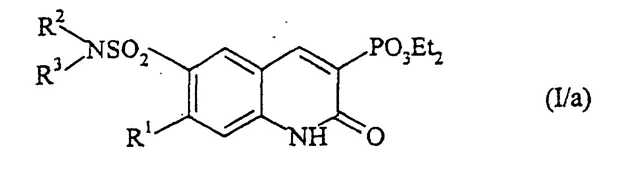 in der R¹, R² und R³ die oben angegebenen Bedeutungen besitzen,
welche teilweise oder vollständig von ihren Schutzgruppen befreit wird in Gegenwart von beispielsweise Trimethylsilanbromid zur Bildung der Verbindung der Formel (I/b), einem Sonderfall der Verbindungen der Formel (I): 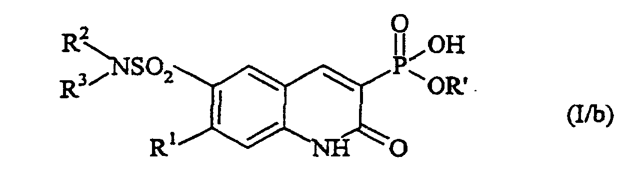 in der R¹, R² und R³ die oben angegebenen Bedeutungen besitzen und R'
ein Wasserstoffatom oder eine Ethylgruppe darstellt,
welche man mit einer Verbindung der Formel (IX):
R" - Cl (IX)
in der R" eine Alkyl-, Aryl-, Arylalkylgruppe oder Gruppe der Formel 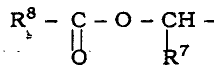 - (worin R⁷ und R⁸ die bezüglich der Formel (I) angegebenen Bedeutungen besitzen) kondensiert,
zur Bildung der Verbindung der Formel (I/c), einem Sonderfall der Verbindungen der Formel (I): 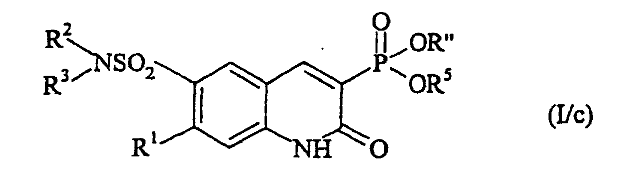 in der R¹, R², R³, R⁵ und R" die oben angegebenen Bedeutungen besitzen,
wobei die Verbindungen der Formeln (I/a) bis (I/c) die Gesamtheit der Verbindungen der Formel (I) darstellen und mit Hilfe einer klassischen Trennmethode gereinigt werden können, welche gewünschtenfalls in ihre Additionssalze mit einer pharmazeutisch annehmbaren Säure oder Base umgewandelt werden können und die man gegebenenfalls mit Hilfe einer klassischen Trennmethode in ihre Isomeren auftrennt.

14. Pharmazeutische Zubereitungen enthaltend als Wirkstoff mindestens eine Verbindung der Formel (I) nach irgendeinem der Ansprüche 1 bis 12 oder eines ihrer Additionssalze mit einer pharmazeutisch annehmbaren Säure oder Base, allein oder in Kombination mit einem oder mehreren pharmazeutisch annehmbaren Trägermaterialien.

15. Pharmazeutische Zubereitungen nach Anspruch 14 für die Herstellung eines Arzneimittels für die akute und chronische Behandlung von pathologischen Phänomenen, die mit der Hyperaktivierung der Neurotransmissionswege für anregende Aminosäuren verknüpft sind, wie Zerebralgefäßvorfällen, Schlaganfällen, Gehirn- oder Rückenmarkstraumata, der Epilepsie, von chronischen neurodegenerativen Erkrankungen, wie der Alzheimerschen Krankheit, der Schizophrenie, der amyotrophischen Lateralsklerose oder der Huntington-Chorea.

## Claims

1. Compounds of formula (I) : 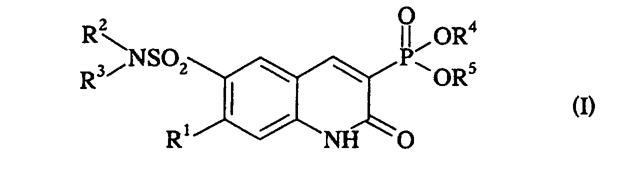 wherein:
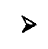 R¹ represents a halogen atom or a trifluoromethyl group,
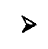 R² represents a hydrogen atom or an alkyl or cycloalkyl group,
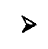 R³ represents an alkyl, cycloalkyl, aryl, arylalkyl, alkoxy, aryloxy, arylalkoxy, hydroxy, aminoalkyl, alkylaminoalkyl, dialkylaminoalkyl, alkylcarbonyl, arylcarbonyl or arylalkylcarbonyl group or an NHR⁶ group (wherein R⁶ represents a hydrogen atom or an alkyl, cycloalkyl, aryl, alkylcarbonyl, arylcarbonyl, arylalkyl or arylalkylcarbonyl group),
or R² and R³ together form with the nitrogen atom carrying them a ring having 5 or 6 carbon atoms, in which one of the carbon atoms can be replaced by an oxygen, nitrogen or sulphur atom or by an SO or SO₂ group or by a NRₐ group (wherein Rₐ represents a hydrogen atom or an alkyl, cycloalkyl, aryl or arylalkyl group),
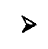 R⁴ and R⁵, which may be identical or different, represent a hydrogen atom or an alkyl, cycloalkyl, aryl or arylalkyl group or a group 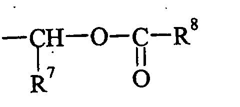 (wherein R⁷ and R⁸, which may be identical or different, represent a hydrogen atom or an alkyl, cycloalkyl or aryl group),
it being understood that:
- "alkyl" is understood to mean a linear or branched alkyl group containing from 1 to 6 carbon atoms,
- "alkoxy" is understood to mean a linear or branched alkoxy group containing from 1 to 6 carbon atoms,
- "cycloalkyl" is understood to mean a cyclic alkyl group containing from 3 to 8 carbon atoms,
- "aryl" is understood to mean the groups phenyl or naphthyl, it being possible for those groups to be unsubstituted or substituted by from 1 to 3 groups selected from alkyl, cycloalkyl, hydroxy, alkoxy, amino, alkylamino, dialkylamino, cyano, nitro, polyhaloalkyl, SO₂NR⁹R¹⁰ (wherein R⁹ and R¹⁰, which may be identical or different, represent a hydrogen atom or an alkyl, cycloalkyl or aryl group) and halogen atoms,
their enantiomers and diastereoisomers and addition salts thereof with a pharmaceutically acceptable acid or base.

2. Compounds of formula (I) according to claim 1 wherein R¹ represents a chlorine atom, their isomers and pharmaceutically acceptable addition salts thereof with an acid or a base.

3. Compounds of formula (I) according to claim 1 wherein R³ represents an alkyl group, their isomers and addition salts thereof with a pharmaceutically acceptable base.

4. Compounds of formula (I) according to claim 1 wherein R³ represents an aryl group, their isomers and pharmaceutically acceptable addition salts thereof with an acid or a base.

5. Compounds of formula (I) according to claim 1 wherein R³ represents a group NHCOR wherein R represents an alkyl or aryl group, their isomers and pharmaceutically acceptable addition salts thereof with an acid or a base.

6. Compounds of formula (I) according to claim 1 wherein R² represents a hydrogen atom, their isomers and pharmaceutically acceptable addition salts thereof with an acid or a base.

7. Compounds of formula (I) according to claim 1 wherein R⁴ and R⁵ simultaneously represent a hydrogen atom, their isomers and pharmaceutically acceptable addition salts thereof with an acid or a base.

8. Compounds of formula (I) according to claim 1 which are 7-chloro-2-oxo-6-[(propylamino)sulphonyl]-1,2-dihydro-3-quinolylphosphonic acid, and 7-chloro-6-[(methylamino)sulphonyl]-2-oxo-1,2-dihydro-3-quinolylphosphonic acid, their isomers and pharmaceutically acceptable addition salts thereof with an acid or a base.

9. Compound of formula (I) according to claim 1 which is 7-chloro-6-[(2-{4-[(dipropylamino)sulphonyl]benzoyl}hydrazino)sulphonyl]-2-oxo-1,2-dihydro-3-quinolylphosphonic acid, its isomers and pharmaceutically acceptable addition salts thereof with an acid or a base.

10. Compound of formula (I) according to claim 1 which is 7-chloro-6-({[2-(dimethylamino)ethyl]amino}sulphonyl)-2-oxo-1,2-dihydro-3-quinolylphosphonic acid hydrobromide, its isomers and pharmaceutically acceptable addition salts thereof with an acid or a base.

11. Compound of formula (I) according to claim 1 which is 6-(anilinosulphonyl)-7-chloro-2-oxo-1,2-dihydro-3-quinolylphosphonic acid, its isomers and pharmaceutically acceptable addition salts thereof with an acid or a base.

12. Compound of formula (I) according to claim 1 which is 6-[(2-benzoylhydrazino)sulphonyl]-7-chloro-2-oxo-1,2-dihydro-3-quinolylphosphonic acid, its isomers and pharmaceutically acceptable addition salts thereof with an acid or a base.

13. Process for the preparation of compounds of formula (I) according to claim 1, **characterised in that** there is used as starting material a compound of formula (II): 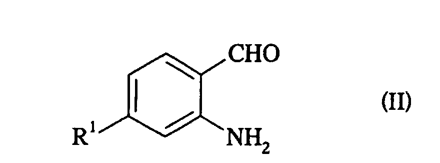 wherein R¹ is as defined for formula (I),
which is condensed, in the presence of a base, such as, for example, pyridine, with a compound of formula (III): 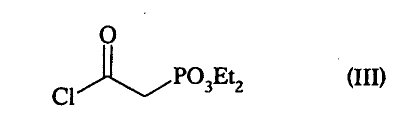 to yield a compound of formula (IV): 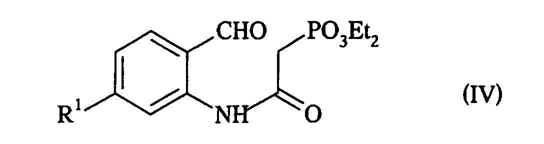 wherein R¹ is as defined hereinbefore,
which is cyclised in the presence of a catalytic amount of piperidine to obtain a compound of formula (V): 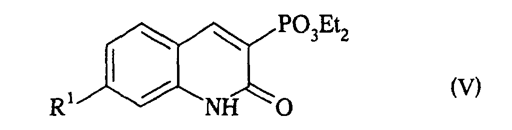 wherein R¹ is as defined hereinbefore,
which is subjected to the action of a mixture of nitric acid and sulphuric acid to yield a compound of formula (VI): 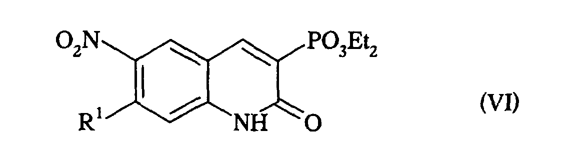 wherein R¹ is as defined hereinbefore,
which is reduced using palladium-on-carbon in the presence of hydrogen or iron in an aqueous alcoholic medium, to yield a compound of formula (VII): 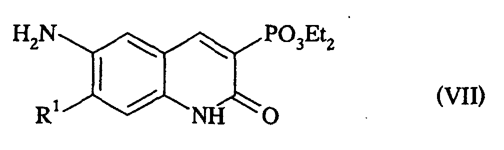 wherein R¹ is as defined hereinbefore,
which is subjected, after conversion to the corresponding diazonium salt, to the action of sulphur dioxide in the presence of CuCl₂ to yield a compound of formula (VIII): 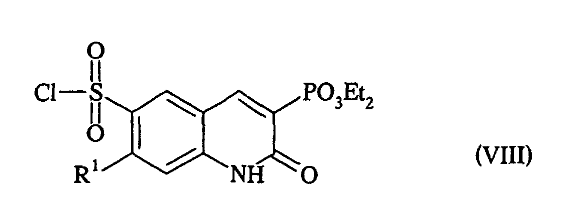 wherein R¹ is as defined hereinbefore,
which is condensed with a compound of formula HNR²R³ wherein R² and R³ are as defined for formula (I) to yield a compound of formula (I/a), a particular case of the compounds of formula (I): 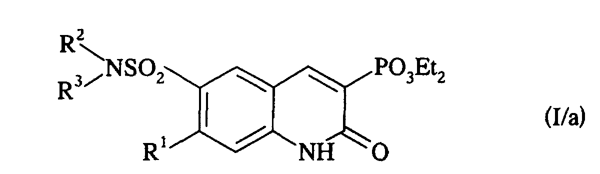 wherein R¹, R² and R³ are as defined hereinbefore,
which is partially or totally deprotected in the presence of, for example, trimethylsilane bromide to yield a compound of formula (I/b), a particular case of the compounds of formula (I) : 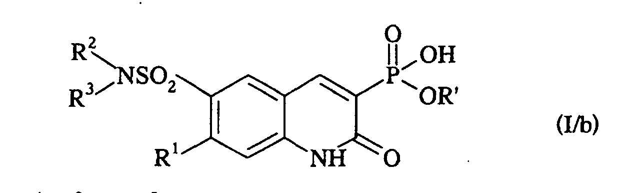 wherein R¹, R² and R³ are as defined hereinbefore and R' represents a hydrogen atom or an ethyl group,
which may be condensed with a compound of formula (IX) :
R"-Cl (IX)
wherein R" represents an alkyl, aryl or arylalkyl group or a group 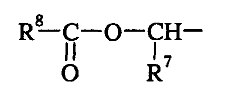 (wherein R⁷ and R⁸ are as defined for formula (I)),
to obtain a compound of formula (I/c), a particular case of the compounds of formula (I) : 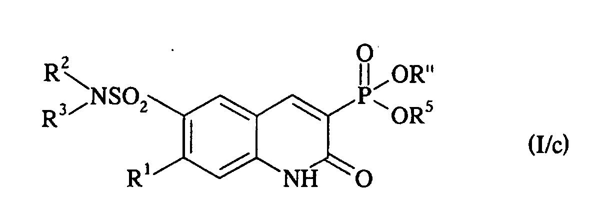 wherein R¹, R², R³, R⁵ and R" are as defined hereinbefore, which compounds of formulae (I/a) to (I/c) constitute the totality of the compounds of formula (I), and can be purified according to a conventional separation technique, are converted, if desired, into their addition salts with a pharmaceutically acceptable acid or base, and separated, where appropriate, into their isomers according to a conventional separation technique.

14. Pharmaceutical compositions comprising as active ingredient at least one compound of formula (I) according to any one of claims 1 to 12 or a pharmaceutically acceptable addition salt thereof with an acid or a base, alone or in combination with one or more pharmaceutically acceptable excipients.

15. Pharmaceutical compositions according to claim 14 for use in the manufacture of a medicament for the acute and chronic treatment of pathological phenomena associated with hyperactivation of the neurotransmission paths to the excitatory amino acids, such as cerebrovascular accident, cerebral or spinal traumatism, epilepsy, chronic neurodegenerative diseases such as Alzheimer's disease, schizophrenia, lateral amyotrophic sclerosis or Huntington's chorea.
